(19) Europäisches Patentamt
European Patent Office
Office européen des brevets

(11) **EP 3 733 654 B1**

(12) **EUROPEAN PATENT SPECIFICATION**

(45) Date of publication and mention
of the grant of the patent:
**24.04.2024 Bulletin 2024/17**

(21) Application number: **20171608.1**

(22) Date of filing: **27.04.2020**

(51) International Patent Classification (IPC):
*C07D 301/02* (2006.01)    *C07D 303/14* (2006.01)

(52) Cooperative Patent Classification (CPC):
**C07D 301/02**

(54) **ONE-STEP SYNTHESIS OF GLYCIDOL FROM GLYCEROL IN GAS-PHASE FIXED-BED CONTINOUS FLOW REACTOR OVER ALKALI METAL PROMOTED ALUMINOSILICATE ZEOLITE CATALYST**

EINSTUFIGE SYNTHESE VON GLYCIDOL AUS GLYCERIN IN EINEM GASPHASEN-FESTBETTREAKTOR MIT KONTINUIERLICHER STRÖMUNG ÜBER EINEN ALKALIMETALLGEFÖRDERTEN ALUMINOSILIKAT-ZEOLITH-KATALYSATOR

SYNTHÈSE EN UNE ÉTAPE DE GLYCIDOL À PARTIR DE GLYCÉROL DANS UN RÉACTEUR À FLUX CONTINU À LIT FIXE EN PHASE GAZEUSE SUR UN CATALYSEUR DE ZÉOLITE D'ALUMINOSILICATE ACTIVÉ PAR UN MÉTAL ALCALIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2019 LU 101204**

(43) Date of publication of application:
**04.11.2020 Bulletin 2020/45**

(73) Proprietor: **Kemijski Institut**
**1000 Ljubljana (SI)**

(72) Inventors:
• **Likozar, Blaz**
**4000 Kranji (SI)**
• **Kostyniuk, Andrii**
**1001 Ljubljana (SI)**
• **Bajec, David**
**8333 Semic (SI)**

(74) Representative: **Weickmann & Weickmann PartmbB**
**Postfach 860 820**
**81635 München (DE)**

(56) References cited:
**WO-A1-2007/132926    US-A1- 2013 090 497**

• **KOSTYNIUK ANDRII ET AL: "One-step synthesis of ethanol from glycerol in a gas phase packed bed reactor over hierarchical alkali-treated zeolite catalyst materials", GREEN CHEMISTRY, vol. 22, no. 3, 10 February 2020 (2020-02-10), pages 753-765, XP055929078, GB ISSN: 1463-9262, DOI: 10.1039/C9GC03262B**
• **Ramli Zainab ET AL: "Effect Of Loaded Alkali Metals On The Structural, Basicity And Catalytic Activity Of Zeolite Beta", Jurnal Teknologi, vol. 42, no. C, 1 June 2005 (2005-06-01), pages 43-55, XP093047818, Retrieved from the Internet: URL:https://www.researchgate.net/profile/Zainab-Ramli-2/publication/44785843_Effect_Of_Loaded_Alkali_Metals_On_The_Structural_Basicity_And_Catalytic_Activity_Of_Zeolite_Beta/links/57d0cb1a08ae601b39a06350/Effect-Of-Loaded-Alkali-Metals-On-The-Structural-Basicity-And-Catalytic-Activity-Of-Zeolite-Beta.pd>**

Remarks:
The file contains technical information submitted after the application was filed and not included in this specification

**Description**

## SUMMARY OF THE INVENTION

The present invention relates to a process for the production of glycidol from glycerol in a fixed-bed continuous flow reactor in the presence of a heterogeneous alkali metal-promoted aluminosilicate zeolite catalyst. The method comprises conversion of glycerol to glycidol at a temperature greater than 250 °C, glycerol concentration 1-60 wt%, total gas hourly space velocity 1000-2500 $h^{-1}$, weight of catalyst 0.5-5.0 g.

## BACKGROUND OF THE INVENTION

Glycerol is the major chemical for future biorefineries. According to recent studies, glycerol was detected as one of the top 12 most important bio-based chemicals in the world. Glycerol has unique substance and can be dehydrated, oxidized, reduced, halogenated, etherified, and esterified to obtain alternative commodity chemicals. The development of novel, selective chemistry to provide new applications for glycerol-derived products remains a key challenge. Therefore, new uses for glycerol need to be found.

Among various commodity chemicals proposed, there are not so many products, which can be obtained in the gas-phase with high conversion and selectivity over stable catalysts. The most interesting products of the gas-phase glycerol valorization over heterogeneous catalysts are allyl alcohol, dihydroxyacetone, hydroxyacetone, acrolein, acrylic acid, lactic acid, 1,2-propanediol, 1,3-propanediol, glycerol carbonate, epichlorohydrin, and glycidol. These products are attractive for industrial commercialization due to many applications and their commercial price on the world market.

Very recently, because of the unique molecule structure, the glycidol received a special attention as a valuable product from glycerol with many potential applications as an important monomer and semi-product in the synthesis of surface-active agents, high-boiling polar solvent, polyurethanes, polyamides, polycarbonates, polyesters, surfactants, lubricating oils, components of cosmetic preparations for skin moisturizing and purifying, hair shampoo, toothpaste, laundering detergents, disinfectants and etc. One of the most important applications of glycidol is the synthesis of analgesic and antiviral drugs, where the latter is the active compound fighting with the human immunodeficiency virus (HIV).

The computational chemistry experiments show that the reaction in gas-phase of glycerol dehydration to glycidol could pass at relatively high temperatures with dehydration mechanisms proposed for these reactions include hydride transfer, pinacol rearrangement, or substitution reactions involving the formation of cyclized products.

Nowadays, there are two the most common methods of glycidol production traditionally used. The first method is epoxidation of allyl alcohol with hydrogen peroxide, and the second is the reaction of epichlorohydrin with a caustic agent. The current problems of industrial production processes of glycidol are following: these methods consist of three stages, allyl alcohol is extremely toxic, epichlorohydrin is made from hydrocarbon feedstocks such as propylene (the raw material source is not renewable), toxic byproducts such as hydrochloric acid, expensive purification steps are necessary.

Recently, glycidol production was realized in liquid-phase from glycerol via glycerol carbonate stage, which is including of a two-step reaction: the synthesis of glycerol carbonate from glycerol and the decarboxylation of glycerol carbonate to glycidol and $CO_2$. The first stage of the glycerol carbonate synthesis can be realized with urea (Y. Endah, M. Kim, J. Choi, J. Jae, S. Lee, H. Lee, Catal. Today 293-294 (2017) 136-141) or dimethyl carbonate (S. Kondawar, C. Patil, C. Rode, ACS Sustain. Chem. Eng. 5 (2017) 1763-1774), in the liquid-phase over different catalysts. However, these processes have many disadvantages such as expensive catalyst, long reaction time, two-step reaction, low feedstock concentration, liquid phase reaction, batch-type reactor, high pressure, solvent, and H-donor are necessary.

In addition, the conversion of glycerol to glycidol that include the three different steps was patented by (A. Pienaar, L. Wilson, M. Stockenhuber, United States Pat. Appl. Publ. US 2013 0090497A1 (2013) 4; A. Pienaar, L. Wilson, M. Stockenhuber, United States Pat. Appl. Publ. US 20140179936A1 (2014) 5). The first step includes the dehydration of glycerol to acrolein over acidic catalyst, such as H-ZSM-5 zeolite catalyst. The second step includes the hydrogenation of acrolein to allyl alcohol over a transition metal catalysts, such as Cd, Ag or Fe supported on a $SiO_2$ or $Al_2O_3$ support. The third step includes the epoxidation of allyl alcohol to glycidol with hydrogen peroxide over a titanium molecular sieve catalyst, such as TS-1, or Au containing catalyst. However, this method has obvious disadvantages, such as three different steps that include three different catalysts with own reaction conditions.

Ramli Zainab et al. (Jurnal Teknologi, vol. 42, no. C, 1 June 2005, pages 43-55) describe the effect of loaded alkali metals on the structural, basicity and catalytic activity of zeolite beta.

The production of glycidol from glycerol was studied more in liquid-phase conditions, mild temperatures, and low concentrations to avoid degradation of the highly reactive substrate. Moreover, the possibility to obtain glycidol from glycerol through the one-step reaction in the industrially more attractive continuous operation rather than in batch mode has not been investigated until now. Comparing with batch reactor the fixed-bed reactor technology is the most efficient reactor type for the synthesis. The fixed-bed continuous flow reactor was chosen by us due to the glycerol conversion and glycidol selectivity can be easily modified with parameters such as gas hourly space velocity (GHSV), temperature reaction, and catalyst weight in comparison with the batch-type reactor.

In this research, we first time investigated the one-step reaction of glycidol synthesis from glycerol in a gas-phase fixed-bed continuous flow reactor. The synthesis of the alkali metal promoted aluminosilicate zeolite catalysts was realized by the incipient wetness impregnation method with the primary focus of this study on the preparation, characterization, and performance of synthesized catalysts.

## SUMMARY OF THE INVENTION

The present invention solves the problems outlined above and provides the method as defined in claim 1. Preferred embodiments of the method are disclosed in claims from 2 to 8, as well as in the following description.

## DETAILED DESCRIPTION OF THE INVENTION

The present invention relates to the use of alkali metal promoted aluminosilicate zeolite heterogeneous catalysts for the direct conversion of glycerol (GL) to glycidol (GLD) in the gas-phase fixed-bed continuous flow reactor. Preferred examples of catalysts are listed in the following: ZSM-5 (HSZ-800-840NHA, HSZ-800-890HOA, CBV 2314, CBV 3024E, CBV 5524G, CBV 8014, CBV 28014), Mordenite (CBV 10A, CBV 21A, HSZ-600-620HOA, HSZ-600-642NAA, HSZ-600-640HOA, HSZ-600-660HOA, HSZ-600-690HOA), USY (HSZ-300-320NAA, HSZ-300-320HOA, HSZ-300-331HSA, HSZ-300-330HUA, HSZ-300-341NHA, HSZ-300-350HUA, HSZ-300-360HUA, HSZ-300-385HUA, HSZ-300-390HUA), Zeolite L (HSZ-500-500KOA), Ferrierite (HSZ-700-720KOA, CP914C), Zeolite Beta (HSZ-900-931HOA, HSZ-900-940HOA, HSZ-900-980HOA, CP814E, CP814C, CP811C-300), Zeolite Y (CBV 100, CBV 300, CBV 400, CBV 500, CBV 600, CBV 712, CBV 720, CBV 760, CBV 780, CBV 901), Cs oxides, $CsNO_3$, $Cs_2SO_4$, CsF, CsCl, CsBr, CsI, $Cs_3PO_4$, $Cs_2CO_3$, $Cs_2C_2O_4$, $CH_3CO_2Cs$ (with varying Cs loading, preferably from 1 to 60), Li oxides, $LiNO_3$, $Li_2SO_4$, LiF, LiCl, LiBr, LiI, $Li_3PO_4$, $Li_2CO_3$, $Li_2C_2O_4$, $CH_3CO_2Li$ (with varying Li loading, preferably from 1 to 60), Na oxides, $NaNO_3$, $Na_2SO_4$, NaF, NaCl, NaBr, NaI, $Na_3PO_4$, $Na_2CO_3$, $Na_2C_2O_4$, $CH_3CO_2Na$ (with varying Na loading, preferably from 1 to 60), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 60), Rb oxides, $RbNO_3$, $Rb_2SO_4$, RbF, RbCl, RbBr, RbI, $Rb_3PO_4$, $Rb_2CO_3$, $Rb_2C_2O_4$, $CH_3CO_2Rb$ (with varying Rb loading, preferably from 1 to 60).

Preferred catalysts are based on the ZSM-5 aluminosilicate zeolite promoted with $CsNO_3$. The suitable amount of alkali metal in the catalysts of the present invention is in the range of from 1 to 60 weight percent relative to the zeolite support. The following examples merely illustrate the invention. It is to be understood that the preferred embodiments described for the catalyst as such of course also are applicable to the use of the catalyst in the process of the present invention.

Examples of suitable alkali metal promoted aluminosilicate zeolite catalysts are xCs-zeolite, xLi-zeolite, xNa-zeolite, xK-zeolite, xRb-zeolite, preferably xCs-zeolite, wherein the alkali metal are supported on a ZSM-5 (HSZ-800-840NHA, HSZ-800-890HOA, CBV 2314, CBV 3024E, CBV 5524G, CBV 8014, CBV 28014), Mordenite (CBV 10A, CBV 21A, HSZ-600-620HOA, HSZ-600-642NAA, HSZ-600-640HOA, HSZ-600-660HOA, HSZ-600-690HOA), USY (HSZ-300-320NAA, HSZ-300-320HOA, HSZ-300-331HSA, HSZ-300-330HUA, HSZ-300-341NHA, HSZ-300-350HUA, HSZ-300-360HUA, HSZ-300-385HUA, HSZ-300-390HUA), Zeolite L (HSZ-500-500KOA), Ferrierite (HSZ-700-720KOA, CP914C), Zeolite Beta (HSZ-900-931HOA, HSZ-900-940HOA, HSZ-900-980HOA, CP814E, CP814C, CP811C-300), Zeolite Y (CBV 100, CBV 300, CBV 400, CBV 500, CBV 600, CBV 712, CBV 720, CBV 760, CBV 780, CBV 901), wherein most preferably ZSM-5. The alkali metals to be employed in accordance with the present invention are preferably employed as oxide, nitrate, sulfate, chloride, phosphate, carbonate, iodate, bromide, fluoride, oxalate, or acetic salts, wherein nitrate salts are most preferably.

The catalysts in accordance with the present invention preferably are aluminosilicate zeolite ZSM-5 and cesium alkali metal supported on this material. The suitable amount of alkali metal in the zeolite support of the present invention is in the range of from 1 to 60 weight percent relative to the support.

The catalysts disclosed herein, in particular the xCs-zeolite, xLi-zeolite, xNa-zeolite, xK-zeolite, xRb-zeolite catalytic system disclosed herein has particularly shown to efficiently gas-phase glycerol conversion in a fixed-bed reactor. Without being bound to the following explanation, it may be that the superior and unexpected activity is attributed to the presence of Lewis and Brönsted acid-base sites, and synergetic interaction between alkali species, preferably Cs species, and zeolite support. The suitable balance between acidity-basicity of the catalyst samples are the key parameter to the high selectivity for the desired glycidol production. In any case, the xCs-ZSM-5 zeolite catalytic system disclosed here shows first time high activity and selectivity of GLD from GL in a gas-phase fixed-bed continuous flow reactor - higher than other catalysts tested or reported in the literature for the gas-phase conversion of GL to GLD. The catalyst according to the present invention shows in the production of GLD from GL in the gas-phase fixed-bed continuous flow reactor conversion of the GL of more than 86 mol% and a yield of GLD of above 40 mol%.

In order to demonstrate the superiority of the present invention, these following catalysts have been evaluated in relation with the catalytic conversion of GL to GLD, including ZSM-5 (HSZ-800-840NHA, HSZ-800-890HOA, CBV 2314, CBV 3024E, CBV 5524G, CBV 8014, CBV 28014), Mordenite (CBV 10A, CBV 21A, HSZ-600-620HOA, HSZ-600-642NAA, HSZ-600-640HOA, HSZ-600-660HOA, HSZ-600-690HOA), USY (HSZ-300-320NAA, HSZ-300-320HOA, HSZ-300-331HSA, HSZ-300-330HUA, HSZ-300-341NHA, HSZ-300-350HUA, HSZ-300-360HUA, HSZ-300-385HUA, HSZ-300-390HUA), Zeolite L (HSZ-500-500KOA), Ferrierite (HSZ-700-720KOA, CP914C), Zeolite Beta (HSZ-900-931HOA, HSZ-900-940HOA, HSZ-900-980HOA, CP814E, CP814C, CP811C-300), Zeolite Y (CBV 100, CBV 300, CBV 400, CBV 500, CBV 600, CBV 712, CBV 720, CBV 760, CBV 780, CBV 901), Cs oxides, $CsNO_3$, $Cs_2SO_4$, CsF, CsCl, CsBr, CsI, $Cs_3PO_4$, $Cs_2CO_3$, $Cs_2C_2O_4$, $CH_3CO_2Cs$ (with varying Cs loading, preferably from 1 to 60), Li oxides, $LiNO_3$, $Li_2SO_4$, LiF, LiCl, LiBr, LiI, $Li_3PO_4$, $Li_2CO_3$, $Li_2C_2O_4$, $CH_3CO_2Li$ (with varying Li loading, preferably from 1 to 60), Na oxides, $NaNO_3$, $Na_2SO_4$, NaF, NaCl, NaBr, NaI, $Na_3PO_4$, $Na_2CO_3$, $Na_2C_2O_4$, $CH_3CO_2Na$ (with varying Na loading, preferably from 1 to 60), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 60), Rb oxides, $RbNO_3$, $Rb_2SO_4$, RbF, RbCl, RbBr, RbI, $Rb_3PO_4$, $Rb_2CO_3$, $Rb_2C_2O_4$, $CH_3CO_2Rb$ (with varying Rb loading, preferably from 1 to 60).

The respective results, some of which are discussed further in relation with experimental data shown below, prove that the catalysts in accordance with the present invention enable the preparation of the desired target molecule in a highly effective manner, thereby overcoming the drawbacks associated with the prior art and solving the problems outlined above.

Accordingly the process in accordance with the present invention comprises the step of converting a starting material, selected GL solution, preferably obtained from renewable resources (bio-based) to GLD in the presence of a heterogeneous catalysts as defined above, preferably selected among catalysts based on zeolite, preferably selected among ZSM-5 (HSZ-800-840NHA, HSZ-800-890HOA, CBV 2314, CBV 3024E, CBV 5524G, CBV 8014, CBV 28014), Mordenite (CBV 10A, CBV 21A, HSZ-600-620HOA, HSZ-600-642NAA, HSZ-600-640HOA, HSZ-600-660HOA, HSZ-600-690HOA), USY (HSZ-300-320NAA, HSZ-300-320HOA, HSZ-300-331HSA, HSZ-300-330HUA, HSZ-300-341NHA, HSZ-300-350HUA, HSZ-300-360HUA, HSZ-300-385HUA, HSZ-300-390HUA), Zeolite L (HSZ-500-500KOA), Ferrierite (HSZ-700-720KOA, CP914C), Zeolite Beta (HSZ-900-931HOA, HSZ-900-940HOA, HSZ-900-980HOA, CP814E, CP814C, CP811C-300), Zeolite Y (CBV 100, CBV 300, CBV 400, CBV 500, CBV 600, CBV 712, CBV 720, CBV 760, CBV 780, CBV 901), preferably ZSM-5, as well as alkali metals, wherein the alkali metals are supported on a ZSM-5, preferably selected among those exemplified above. As for the catalyst, as such the alkali metals to be employed in accordance with the present invention are preferably selected among Li, Na, K, Rb, Cs, most preferably Cs. These alkali metals are preferably employed as compounds selected among oxide, nitrate, sulfate, chloride, phosphate, carbonate, iodate, bromide, fluoride, oxalate, acetate salts, wherein nitrate salts are preferred.

The starting materials to be converted in accordance with the present invention typically are provided in a polar solvent, preferably water. It has been found that the concentration of the starting material in the polar solvent preferably is at least 1 wt% and not more than 60 wt%, preferably 10 to 35 wt%. It has been found that the amount of catalyst in the gas-phase reaction in fixed-bed continuous flow reactor is not less than 0.5 g and not higher than 5.0 g.

The gas hourly space velocity is in the range of from 1000-1250 $h^{-1}$. A preferred combination of reaction conditions is a temperature of from 330 to 360 °C and a gas hourly space velocity of from 1000 to 1250 $h^{-1}$. The reaction is carried out with a carrier gas, which is nitrogen, due to its abundance and low cost.

The process disclosed here is carried out at a temperature greater than 250 °C, typically at a temperature from 250 °C to 450 °C, preferably from 300 to 350 °C, more preferably from 330 to 360 °C, such as 350 °C. The reaction may be carried out in any suitable reactors known to the skilled person, but stainless steel reactors (fixed-bed continuous flow reactor) are in particular suitable.

The catalyst used herein is capable to show in the production of GLD from GL in the gas-phase fixed-bed continuous flow reactor conversion of the GL of more than 86 mol% and a yield of GLD of above 40 mol%.

It is to be understood, that the various process parameters, such as starting material concentration, gas-hourly space velocity, catalyst amount, temperature, while being disclosed individually, are considered in the context of the present invention in combination, such as a process employing water as solvent, a starting material concentration of from 1 to 60 wt%, a catalyst amount of from 0.5 to 5.0 g, a reaction temperature of from 250 to 450 °C, and a the gas hourly space velocity of from 1000 to 2500 $h^{-1}$. However, all other combinations possible from the ranges and preferred embodiments are also comprised by and disclosed in this invention.

In the examples described herein, the catalytic conversion of glycerol was performed using a stainless steel fixed-bed continuous flow reactor under the standard operation conditions. The novel process of the present invention was carried out at a temperature greater than 250 °C, most preferably between 250 and 450 °C, especially between 330 and 360°C. The reactions were carried out under atmospheric pressure. In general, the reaction should be conducted under conditions where the gas hourly space velocity of the feedstock solution over the catalyst is appropriate to generate the desired products. The gas hourly space velocity is in the range of between 1000-1250 $h^{-1}$.

The invention is further illustrated in the following figures and examples concerning preferred embodiments. However, they are not to be understood as limiting the subject matter of the invention.

## FIGURES

**Fig. 1.**    illustrates the setup used for the continuous gas-phase conversion of glycerol to glycidol. 1 - $N_2$ gas storage tank; 2 - inlet glycerol tank; 3 - HPLC pump; 4 - convection heater, 5 - hot box; 6 - reactor; 7 - oven; 8 - packed-bed; 9 - quartz wool; 10 - steel porous plate; 11 - six port bypass valve; 12 - Peltier cooling cell; 13 - GC (Agilent GC-7890A) with FID detector and GC-MS (Shimadzu, QP2010); 14 - personal computer.

**Fig. 2.**    Proposed reaction pathways from glycerol to glycidol. This scheme contains all main products during glycerol conversion over synthesized catalysts. Allyl alcohol can be obtained directly from glycerol dehydration and transfer hydrogenation mechanism.

**Fig. 3**    Temporal evolution of selectivity for main reaction products (glycidol, hydroxyacetone, allyl alcohol) and glycerol conversion (GL conv.) over the 20CsZSM-5(30) - (a); 20CsZSM-5(1500) - (b).

**Fig. 4.**    HRSEM images of the A - HZSM-5(30), B - 20CsZSM-5(30), C - HZSM-5(1500), D-20CsZSM-5(1500).

**Fig. 5.**    HRTEM image and elements mapping of the 20CsZSM-5(1500) zeolite catalyst - (A, B).

**Fig. 6.**    TGA profiles for the 20CsZSM-5(30) - (a) and 20CsZSM-5(1500) - (b) fresh and spent (TOS = 27 h) catalyst samples.

**Fig. 7.**    XRD patterns of the HZSM-5(1500), 20CsZSM-5(1500), and $CsNO_3$. The reference lines on the 2θ axis correspond to the materials present and were obtained from the COD database.

**Fig. 8.**    $N_2$-adsorption and desorption isotherms (a); BJH pore size distribution (b) of the catalyst samples.

**Fig. 9.**    $CO_2$-TPD profiles of HZSM-5(30); HZSM-5(1500); 20CsZSM-5(30); 20CsZSM-5(1500) catalysts.

**Fig. 10.**    $NH_3$-TPD signals of the HZSM-5(30), HZSM-5(1500), 20CsZSM-5(30) and 20CsZSM-5(1500) catalysts as a function of temperature.

**Fig. 11.**    FTIR spectra of pyridine chemisorbed on parent HZSM-5(30) and HZSM-5(1500) zeolites before and after modification with Cs.

**Fig. 12.**    GC-FID traces for a typical run over the 20CsZSM-5(1500) catalyst. Rxn conditions: catalyst, 0.5 g, 250-400 $\mu$m; TOS = 3 h; $T_{reactor}$ = 350 °C; C(GL) = 10 wt%; FR(GL) = 2.0 ml/h; $GHSV_{total}$ = 1250 $h^{-1}$.

## EXAMPLES

The following examples are included to further illustrate various embodiments of the presently disclosed subject matter.

## EXAMPLE 1

**Materials used**

In the next examples, the following starting materials were used: ZSM-5 (HSZ-800-890HOA, CBV 3024E,), $CsNO_3$ (Sigma-Aldrich, 99%), glycerol (Sigma-Aldrich, 99%), nitrogen (5.0, Messer, Bad Soden am Taunus, Germany), $NH_3$ (10 vol% in He, Linde, Pullach, Germany), helium (5.0, Messer, Bad Soden am Taunus, Germany) and carbon dioxide (10 vol% $CO_2$ in He, Linde, Pullach, Germany), glycidol (Sigma-Aldrich, 96%), acrolein (Fluka, >95%), allyl alcohol (Sigma-Aldrich, >99%), hydroxyacetone (Alfa Aesar, 95%), 2-methoxy-1,3-dioxalane (Sigma-Aldrich, >99%), glycerol formal (Sigma-Aldrich, 98%), acetaldehyde (Sigma-Aldrich, >99.5%), propionaldehyde (Sigma-Aldrich, 97%), acetic acid (Sigma-Aldrich, >99.7%), acetone (Sigma-Aldrich, >99.5%), propionic acid (Sigma-Aldrich, >99.5%), acrylic acid (Sigma-Aldrich, 99%), 1,2-propanediol (Sigma-Aldrich, >99%), n-propanol (Sigma-Aldrich, 99.5%), formaldehyde (Sigma-Aldrich, 37wt% in $H_2O$), methanol (Sigma-Aldrich, >99.9%).

## EXAMPLE 2

### Catalysts preparation

The preparation of the alkali metal promoted aluminosilicate zeolite catalysts has involved the impregnation of zeolite support using the wet impregnation method with solutions 0.01 M of alkali metal. The zeolite support were calcined at 550 °C in air for 6 h to convert the powder from the ammonium form to its protonated form and/or to completely remove any organic residues from the protonated form. The alkali metal solution was added to the corresponding zeolite with vigorous stirring and 80 °C. Stirring continued until suspension turned into a slurry. Then samples were dried overnight at 110 °C, and calcined at 550 °C for 6 hours.

## EXAMPLE 3

### Catalytic activity performance

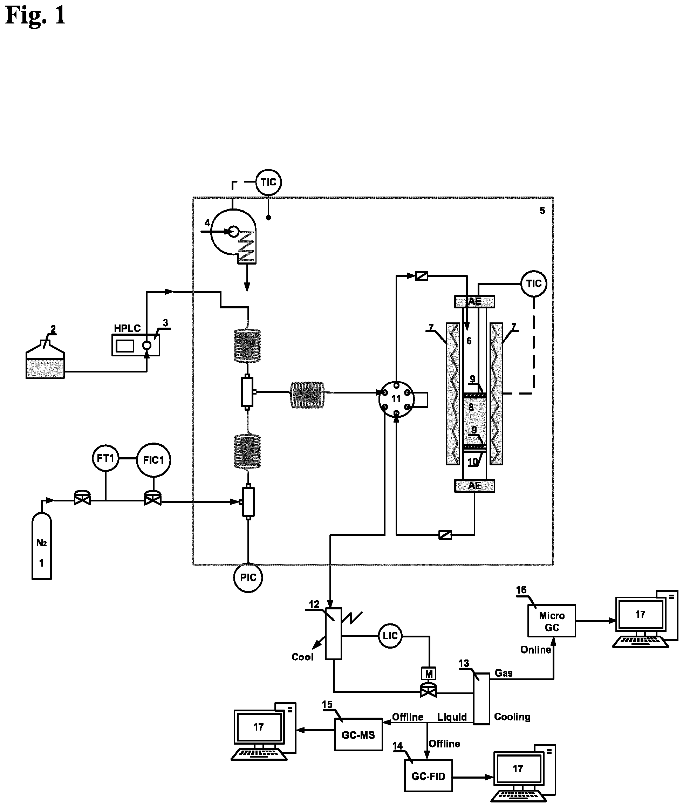

The gas-phase conversion of GL into GLD (Fig. 1) was studied in an automated laboratory fixed-bed microactivity reactor (Microactivity-Reference, PID Eng&Tech). 0.5 g of the alkali metal promoted aluminosilicate zeolite catalyst was placed in a packed bed tubular reactor and stabilized with glass wool on both sides. The glycerol of 1-60 wt% aqueous solution was kept at room temperature in a reservoir tank (3) outside of the hot box. It was injected into the hot box with a 307 HPLC Piston Pump (4) (Gilson Inc.). The $N_2$ gas was introduced into the hot box (6) with mass flow controllers (FT1). The glycerol aqueous solution was entered to the hot box (6) was vaporized, since the inside temperature was held at 140 °C, with the help of an electric convection heater (5), controlled by the temperature controller, TIC.

Thereafter it was mixed of the glycerol solution vapour and was introduced $N_2$ gas. The inlet stream was then passed through a six-port VICI reactor standard bypass valve (12) and was either introduced into the reactor (7) or sent out of the reactor. The reactor was a stainless steel tube (9 mm internal diameter, 305 mm long). The catalyst was packed inside of the tube (9) and insulated with a 15 mm layer of glass wool at the top and bottom (10).

The temperature of the packed-bed was measured with a thermocouple with the TIC controller. After leaving the reactor tube, the outlet stream was conducted through the six-port bypass valve (12) and into the Peltier cell (13). The product was cooled down inside the internal storage. The liquid level in the storage was controlled with a level controller (LIC) and the excess product was sent into absorber placed below the reactor. The collected liquid samples were then analyzed by gas chromatography GC (Agilent GC-7890A) with measurement repeatability of the relative standard deviation (RSD) $\leq$ 5% equipped with FID detector. Hydrogen used as carrier gas with a flow rate of 1 mL/min. The capillary column was 30m $\times$ 0.25mm $\times$ 0.25$\mu$m DB-WAX Ultra Inert. To achieve effective product separation, the column was held at 40 °C for 4 min before the temperature was ramped up to 200 °C at a rate of 12 °C/min and remained there for 10 min. The split ratio was 1:150.

All products were quantified via an external calibration method verified by a gas chromatography-mass spectrometry system (Shimadzu, GC/MS-QP2010) with the capillary column 30m $\times$ 0.25mm $\times$ 0.5um Zebron ZB-WAX-Plus. All gas samples were analyzed online by micro GC Fusion Inficon with Rt-Molsieve 5A, Rt-Q-BOND and Rt-U-BOND capillary columns.

Calibration curves were measured in the 0.1-10 wt% range by using chemicals mentioned above in the chemical used paragraph. The conversion of glycerol, $X_{GL}$, and the selectivity to the product $i$, S$i$, calculated using the equations below, where $n$ refers to the moles of compound and in/out to the reactor inlet/outlet.

$$X_{GL}\left(mol\%\right) = \frac{\left(n_{GL}^{in} - n_{GL}^{out}\right)}{n_{GL}^{in}} \times 100\;;$$

$$S_i\left(mol\%\right) = \frac{n_i}{n_{GL}^{in} - n_{GL}^{out}} \times \frac{Z_i}{Z_{GL}} \times 100\;;$$

$$Y_i\left(mol\%\right) = \frac{n_i}{n_{GL}^{in}} \times \frac{Z_i}{Z_{GL}} \times 100\;;$$

where $n_{GL}^{in}$ and $n_{GL}^{out}$ are the input and output flows of glycerol (mol/min), $n_i$ is the flow of the products i (mol/min), $Z_{GL}$ = 3 (the number of carbon atoms in the GL molecule), and $Z_i$ represents the number of carbon atoms in the products. The values in this article are in mole percent. The mass balance was calculated for each experiment and was usually above 92%, where the difference in mass balance was in the range of the experimental error. The studied conversion and selectivity figures were calculated by the values of three measurements.

**EXAMPLE 4**

**Product distribution over cesium promoted ZSM-5(1500) zeolite catalysts in the gas-phase reaction of GL to GLD**

The conversion of GL to GLD has been performed in the same conditions as those described in the example above (Catalyst evaluation paragraph). The results obtained with over of the catalysts of formula xCs-ZSM-5(1500) are given in Table 1 below. The number in parenthesis is the $SiO_2/Al_2O_3$ ratio.

**Table 1.** Product distribution data over cesium promoted ZSM-5 zeolite catalysts in the gas-phase glycerol conversion to glycidol.

| Catalyst | Glycidol selectivity (mol%) | Hydroxyacetone selectivity (mol%) | 1,2-Propanediol selectivity (mol%) | Acrolein selectivity (mol%) | Allyl alcohol selectivity (mol%) | Others (mol%) | Time-on-stream (h) |
|---|---|---|---|---|---|---|---|
| 10Cs-ZSM-5 (1500) | 42.9 | 38.7 | 3.8 | 5.6 | 1.5 | 7.5 | 27 |
| 20Cs-ZSM-5 (1500) | 64.3 | 28.1 | 2.8 | 0.5 | 2.7 | 1.6 | 27 |
| 40Cs-ZSM-5 (1500) | 56.2 | 32.1 | 5.2 | 1.8 | 4.7 | 0 | 27 |

**EXAMPLE 5**

**Catalytic conversion of GL to GLD over cesium promoted ZSM-5(1500) zeolite catalysts in a fixed-bed continuous flow reactor**

These results show the glycidol yield and glycerol conversion in the same conditions as those of example 6. The results are tabulated in Table 2 below. The number in parenthesis is the $SiO_2/Al_2O_3$ ratio.

**Table 2.** Gas-phase glycerol conversion and glycidol yield over cesium promoted ZSM-5(1500) zeolite catalysts in a fixed-bed continuous flow reactor

| Catalyst | Glycidol yield (mol%) | Glycerol conversion (mol%) | Time-on-stream (h) |
|---|---|---|---|
| 10Cs-ZSM-5(1500) | 12.5 | 32.9 | 3 |
| 20Cs-ZSM-5(1500) | 40.4 | 86.3 | 3 |
| 40Cs-ZSM-5(1500) | 4.7 | 21.8 | 2 |

**EXAMPLE 6**

**The reaction pathway**

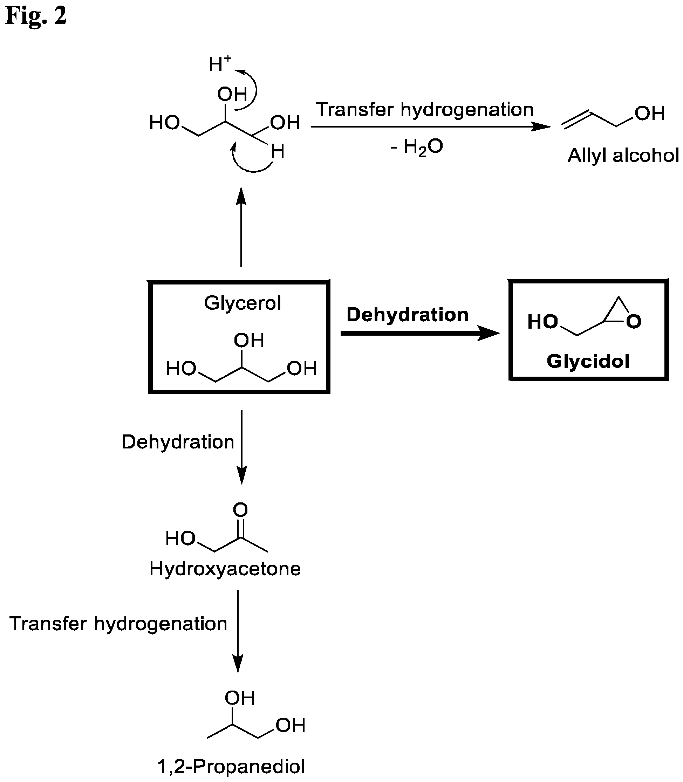

The pioneering gas-phase conversion of glycerol into glycidol in one-step over studied catalysts was achieved. Fig. 2 presents a possible reaction pathway for glycerol conversion by intramolecular dehydration and transfer hydrogenation reactions with the participation of basic sites.

We suggest that glycidol was produced directly through the intramolecular dehydration of glycerol over studied catalysts. In our experimental work the ZSM-5(1500) zeolite catalysts with $SiO_2/Al_2O_3$ = 1500 and 30 were utilized in the gas-phase conversion of glycerol.

## EXAMPLE 7

### Temporal evolution of selectivity for main reaction products and glycerol conversion over the 20CsZSM-5(30) and 20CsZSM-5(1500) catalysts

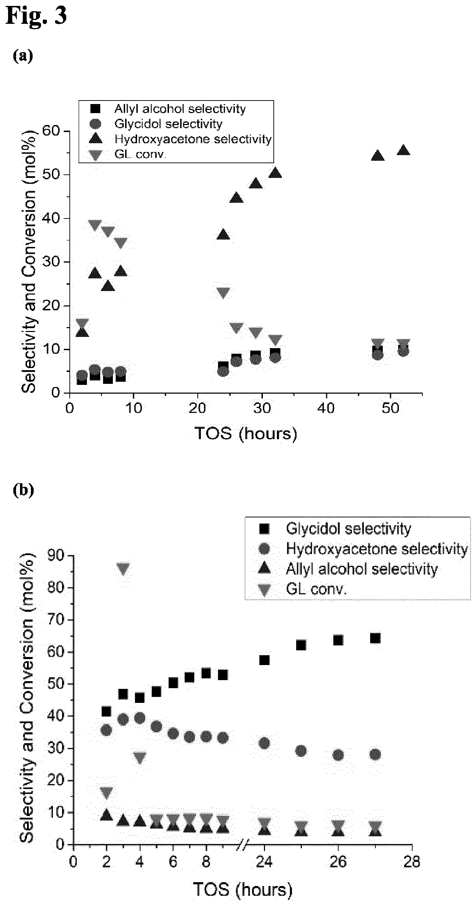

We have studied $CsNO_3$ promoted ZSM-5(1500) and ZSM-5(30) with $SiO_2/Al_2O_3$ = 1500 and 30 at 350 °C (Fig. 3). Glycidol, hydroxyacetone, allyl alcohol, 1,2-propanediol, acrolein, acetaldehyde, acetic acid, and propionic acid are liquid products which were identified. It was found that glycidol appeared only over Cs-ZSM-5(1500) catalyst as the main product and its selectivity was increasing continuously during the 27 h time on stream.

The highest conversion achieved was 86.3% over 20CsZSM-5(1500) catalyst within 3 h time-on-stream, but during the prolonging of reaction time, the conversion was drastically decreased due to the coke formation. At the same time, 20CsZSM-5(30) catalyst shows hydoxyacetone as a main product. Only low traces of acrolein was identified (it was not shown in Fig. 3 because of the low concentration of the latter).

The catalysts were characterized by XRD, $N_2$ physisorption, STEM-EDX, SEM, pyridine-DRIFT, $CO_2$-TPD, $NH_3$-TPD, and TGA techniques. Some additional examples are presented below.

## EXAMPLE 8

### Scanning electron microscopy (SEM)

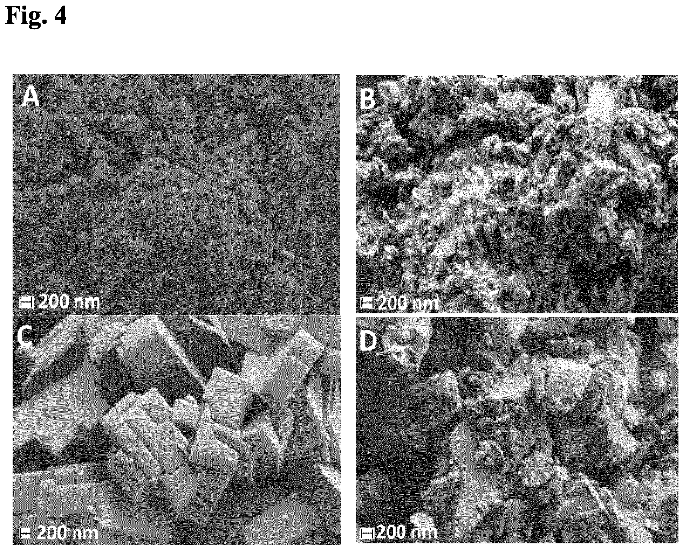

The SEM images in Fig. 4 provide a view on the morphologies and particles sizes of the parent and CsNOs impregnated zeolites. Fig. 4A and B show the particles size less than 50 nm for the HZSM-5(30) and 20CsZSM-5(30) catalysts, while HZSM-5(1500) and 20CsZSM-5(1500) samples exhibit the particles size in the range of 1-2 $\mu$m (Fig. 4C and D). As can be seen from the SEM images, all crystalline zeolites are agglomerated and composed of cube nanocrystals. Also, it is evident from the microphotographs that the framework of the zeolite support has suffered from alkali dissolution after $CsNO_3$ impregnation (Fig. 4B and D).

## EXAMPLE 9

### Scanning transmission electron microscopy with energy dispersive X-ray spectroscopy (STEM-EDXS) analysis

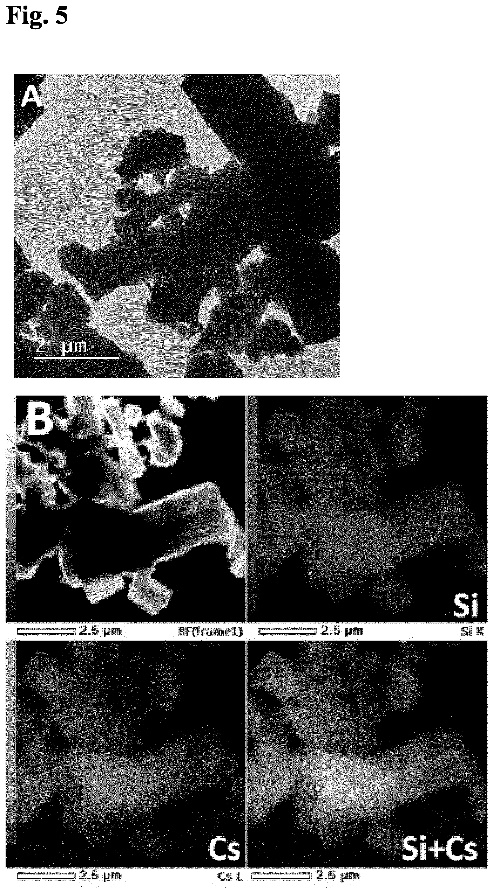

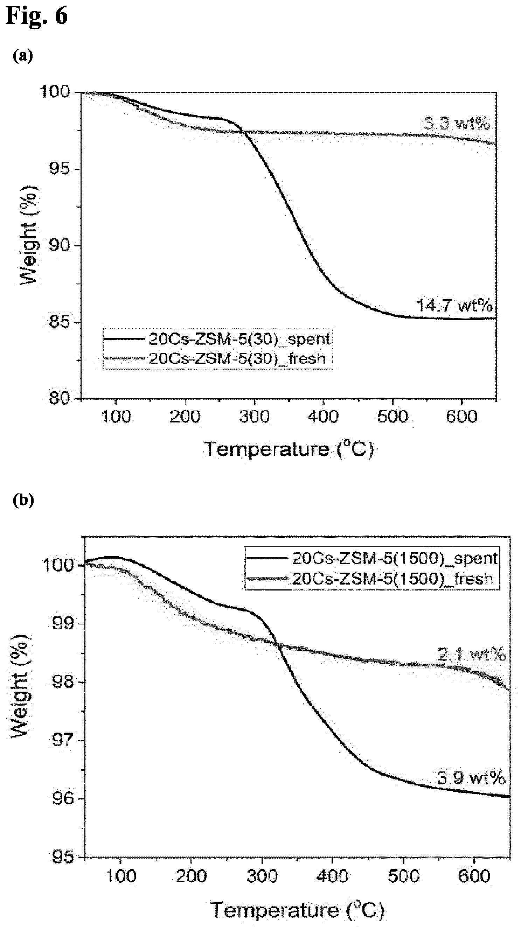

Fig. 5 shows the TEM images and elements mapping of the 20CsZSM-5(1500) catalyst. It is clear that Cs species are homogeneously distributed into the zeolite framework and most of these species are located inside of the zeolite crystals (Fig. 5B).

## EXAMPLE 10

### Thermogravimetric analysis (TGA)

Thermal analyses were performed on the catalysts after catalytic tests. Temperature ramp was set up to 650 °C. TGA was used to determine the amount of coke deposited on the catalyst samples after catalytic tests for 27 h under the same experimental conditions. The initial weight loss of the samples was observed in the temperature range of 50 to 250 °C (Fig. 6). This weight decrease can be related to the evaporation of physisorbed, chemisorbed water and volatile species. The major losses of weight were observed above 255-280 °C for both samples due to the presence of coke.

It was shown (Fig. 6) that the highest coke formation was 14.7 wt% over 20CsZSM-5(30) catalyst, which can be related to the lower $SiO_2/Al_2O_3$ ratio and the acid sites presence. The 20CsZSM-5(1500) catalyst showed a high coke resistance with the mass loss of 3.9 wt% in comparison to the 20CsZSM-5(30) sample. This observation can be explained by the high $SiO_2/Al_2O_3$ ratio of zeolite support and a complete disappearance of acid sites.

We have found (Fig. 6) that TGA profiles of the 20CsZSM-5(30) and 20CsZSM-5(1500) fresh catalysts exhibited much lower weight decrease due to the absence of coke deposition. At the same time, it was observed on TGA profiles the decreasing trend of mass sample with increasing of temperature-programmed heating mode. These TGA results can be explained by the loosing of water molecules at a low temperature. At the higher temperature the catalyst weight decreasing is attributed to partial decomposition of $CsNO_3$, which is happens in the temperature range 400-750 °C.

## EXAMPLE 11

**Powder X-ray diffraction (XRD) analysis**

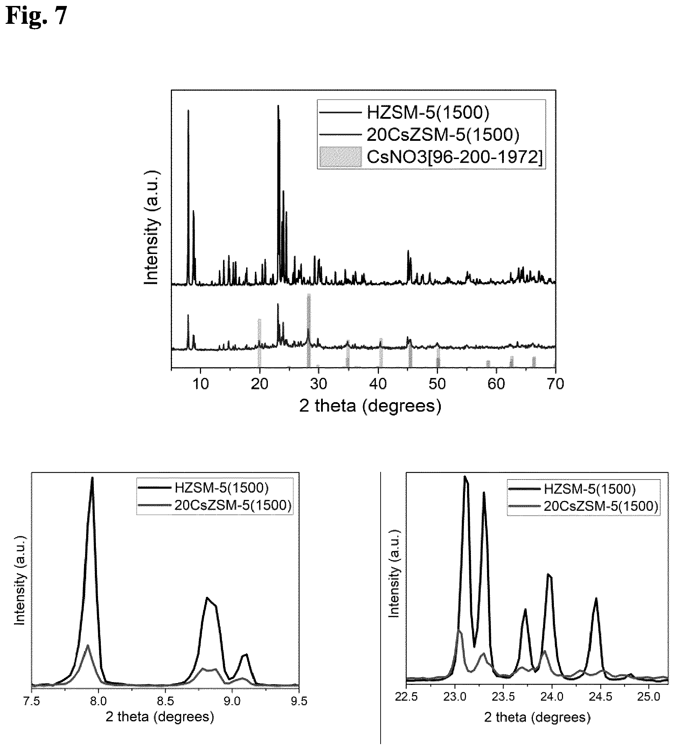

Fig. 7 shows the XRD patterns of the HZSM-5 and CsZSM-5 catalysts with $SiO_2/Al_2O_3$ molar ratio of 30 and 1500. The intensity of diffraction peaks assigned to MFI framework was changed and also the lattice parameters were modified after alkali-treatment.

The peaks ascribed to $CsNO_3$ are observed in Fig. 7 for the 20CsZSM-5(1500) catalysts at around 2θ = 19.8, 28.2, 34.8, 40.4, 45.4, 50.0, 62.3, 66.3 and 69.9° (COD № 96-200-1972). The peaks related to HZSM-5 framework are decreasing with the loading of $CsNO_3$ to HZSM-5. As shown on the XRD diffractograms this CsNOs addition has an impact on the framework of HZSM-5 which is also supported by SEM results above. In Fig. 6 patterns represent distinct sharp diffraction peaks in 2θ ranges of 7.5-9.5 and 22.5-25.2°, where all samples exhibit the typical diffraction peaks of HZSM-5 original structure. The XRD patterns of the studied catalysts showed that the intensity of the Si diffraction peaks at 2θ = 7.9°, 8.8°, 23.0°, 23 3 °, 23.7°, 23.9°, 24.4° have decreased after addition of $CsNO_3$ compared with parent zeolite catalysts with $SiO_2/Al_2O_3$ = 1500.

## EXAMPLE 12

**$N_2$-adsorption and desorption isotherms and BJH (Barrett-Joyner-Halenda) pore size distribution studies**

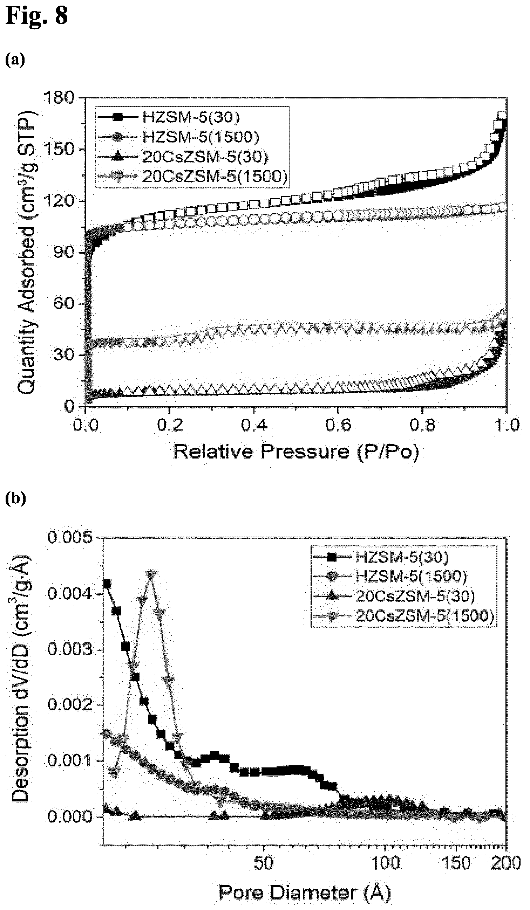

Presented results in Fig. 8 show that the incorporation of Cs metal species to the parent HZSM-5(1500) zeolites decreased the BET surface areas, microporous and mesoporous surface areas, and pore volumes. Only the pore diameter was increased in the case of 20CsZSM-5(30) catalyst compared to the HZSM-5(30), namely from 6.4 to 24.6 nm. This observation can be explained by the higher dissolution of zeolite frameworks caused by interaction between alkali metal - zeolite support with lower $SiO_2/Al_2O_3$ ratio. In addition, the crystallite size, calculated from different individual reflections in XRD patterns by the Scherrer equation, exhibited a decrease from 91.5 to 78.7 nm for the CsZSM-5(1500) catalyst. Zeolites crystallite size decreasing can lead to improvement of catalytic activity and selectivity. At the same time, CsZSM-5(30) catalyst showed negligible trend of the crystallite size increase from 57.4 to 61.6 nm. The crystallinity of the CsZSM-5(30) and CsZSM-5(1500) catalysts was decreased from 100 to 33.4% and 34.6%, respectively. These results can be explained by desilication of ZSM-5 zeolite framework (Fig. 8).

## EXAMPLE 13

**$CO_2$-temperature-programmed desorption ($CO_2$-TPD) analysis**

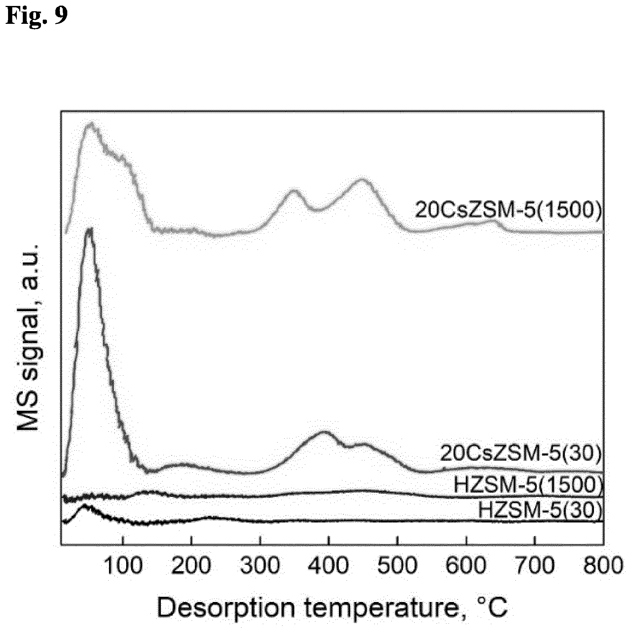

Fig. 9 shows the $CO_2$-TPD results of the parent HZSM-5 and alkali-treated zeolites. It was found that $CsNO_3$ impregnated zeolites have relatively high basicity. The small desorption peak at the temperature of 45 and 135 °C is present for the HZSM-5(30) and HZSM-5(1500) zeolites, respectively. These peaks indicate on the presence of surface basic sites that was due to the interaction between the framework of HZSM-5 and $CO_2$. It is known that zeolites can include acid and basic sites, where the latter can appear from the negative charge in the Si-O-Al species. Zeolites basicity depends on their chemical composition (on framework and nonframework atoms) and the structure type influences the oxygen basicity. ZSM-5 alkali-promoted zeolites showed basic properties due to a high charge on oxygen, which relates to $Cs^+$ cation with the lowest electronegativity (0.79) and thus, weak acidity.

## EXAMPLE 14

**$NH_3$-temperature-programmed desorption ($NH_3$-TPD) analysis**

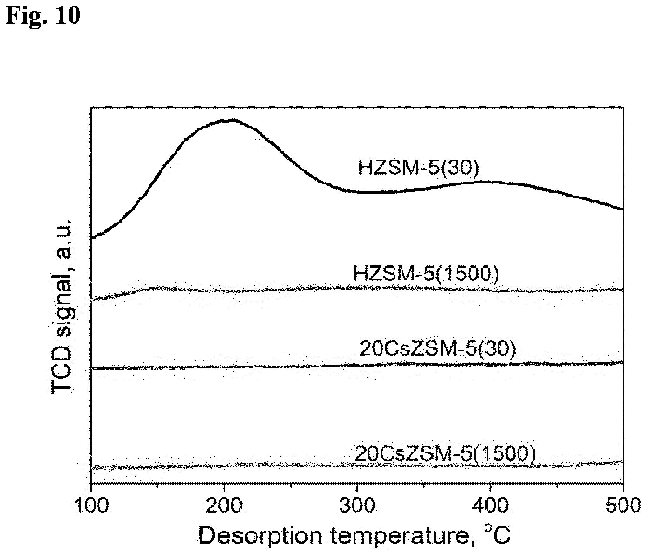

Fig. 10 shows the $NH_3$-TPD signals of the HZSM-5 and CsZSM-5 zeolite catalysts. The acidic sites for the Cs promoted zeolite catalysts were not detected by $NH_3$-TPD analysis. The $NH_3$-TPD profile clearly indicates that when $CsNO_3$ was loaded onto the zeolite framework, the surface acidity decreased (or totally disappeared) but the surface basicity increased, which is also in agreement with previous reports.

The 20CsZSM-5(30) and 20CsZSM-5(1500) samples exhibit three main desorption peaks with representing weak (45-55 °C), medium (350-450 °C) and strong (≈ 640 °C) basic sites. These data clearly indicate that the addition of $CsNO_3$ to HZSM-5 increase the intensity of the $CO_2$ desorption peak and improve the surface basicity, which is in good agreement with the literature. The total basicity increased from 0.9 to 18.2 $\mu mol_{CO2}/g_{catalyst}$ after $CsNO_3$ impreg-

nation to HZSM-5(30) zeolite and from 1.1 to 17.3 $\mu mol_{CO2}/g_{catalyst}$ when HZSM-5(1500) zeolite support was impregnated.

At the same time, HZSM-5(30) catalyst exhibits usual desorption spectrum with two distinct desorption peaks of the $NH_3$-TPD profile containing a low-temperature peak at 200 °C related to weak acid sites and a high-temperature peak at 400 °C corresponding to strong acid sites. It was shown that with the rising $SiO_2/Al_2O_3$ ratio of HZSM-5 zeolites from 30 to 1500, the acid sites almost completely disappeared. This shows that HZSM-5(1500) zeolite has a very weak acidic character. Only a small peak was detected at 150 °C, which is related to weak acid sites and also correlated with Pyr-DRIFT analysis below.

**EXAMPLE 15**

**Pyridine diffuse reflectance infrared Fourier transform spectroscopy (DRIFTS) analysis**

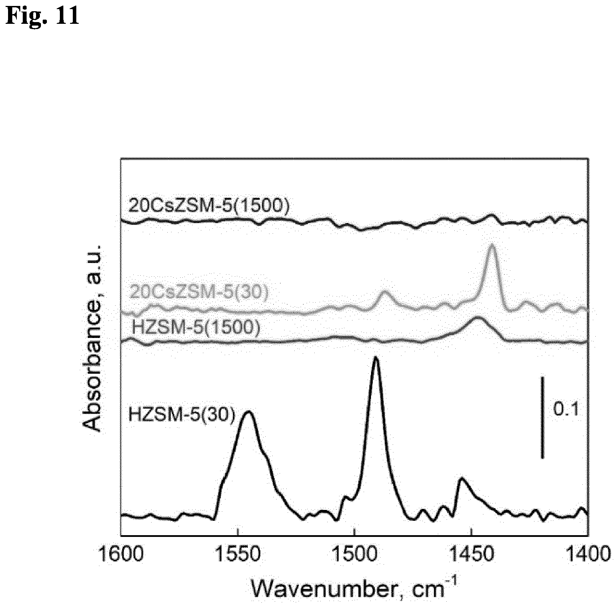

On Fig. 11, the effect of 20 wt% Cs addition on the acidity of HZSM-5(30) and HZSM-5(1500) zeolites is studied by Pyr-DRIFT technique. Acidity in CsZSM-5(1500) is totally lost, whereas in the case of aluminum-rich CsZSM-5(30) the BAS are totally eliminated and LAS are increased approximately in 2 times: from 0.13 to 0.23 mmol/g.

The LAS peak of 20CsZSM-5(30) shifts to lower frequencies, indicating weakening of its total acidity (0.23 mmol/g) compared to pristine HZSM-5(30) with 0.63 mmol/g. This increasing of LAS over the 20CsZSM-5(30) catalyst can be explained by the weak Lewis acidity of $Cs^+$ in combination with alumina interaction. This increase of LAS was also observed in the literature, when the loading of $CsNO_3$ was above 10 wt%. The absence of LAS over the 20CsZSM-5(1500) catalyst can be attributed to the very low content of alumina in the HZSM-5(1500) zeolite support.

**EXAMPLE 16**

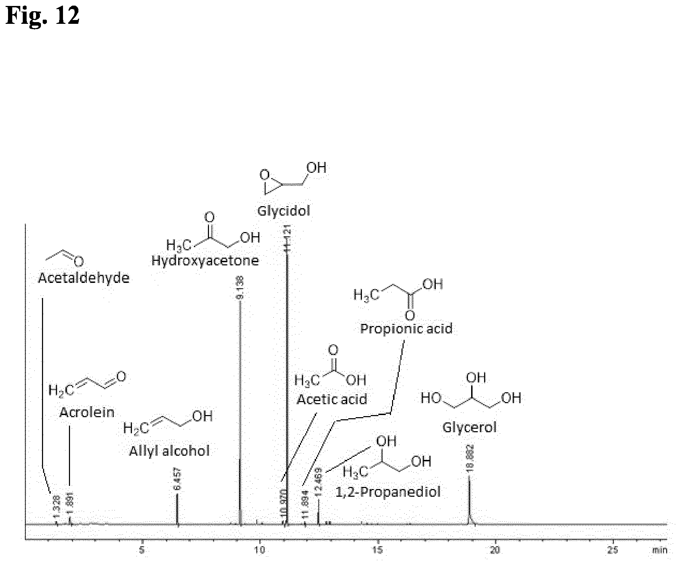

GC-FID traces for a typical run over the 20Cs-ZSM-5 catalyst shows on the Fig. 12 the formation of GLD, hydroxyacetone, allyl alcohol, 1,2-propanediol, acrolein, acetaldehyde, acetic acid, and propionic acid liquid products formation with $GHSV_{total}$ = 1250 h$^{-1}$. These products approximately covered more than 96% from the total mass.

**Claims**

1. Process for the production of glycidol (GLD) comprising the steps:

   **(a)** feeding a starting material over a heterogeneous catalyst into a fixed-bed continuous flow reactor,
   **(b)** heating said mixture at a temperature greater than 250°C to obtain glycidol,
   **(c)** flowing $N_2$ as a carrier gas and glycerol (GL) as a feed with total gas hourly space velocity 1000-2500 h$^{-1}$, and
   **(d)** collecting the GLD product,
   wherein the starting material is GL solution,
   wherein the catalyst comprises at least one alkali metal compound in an amount of 1 to 60 wt% supported on aluminosilicate zeolite.

2. The process according to claim 1, wherein the concentration of GL in the solution is in the range from 1 to 60 wt%.

3. The process according to any one of the preceding claims, wherein the alkali metals are selected from lithium (Li), sodium (Na), potassium (K), rubidium (Rb), and cesium (Cs).

4. The process according to any one of the preceding claims, wherein the zeolite is selected from the group consisting of ZSM-5, Mordenite, USY, Zeolite L, Ferrierite, Zeolite Beta, and Zeolite Y, preferably ZSM-5.

5. The process according to any one of the preceding claims, wherein the alkali metal compounds are selected from the group consisting of Cs oxides, $CsNO_3$, $Cs_2SO_4$, CsF, CsCl, CsBr, CsI, $Cs_3PO_4$, $Cs_2CO_3$, $Cs_2C_2O_4$, $CH_3CO_2Cs$ (with varying Cs loading, preferably from 1 to 60), Li oxides, $LiNO_3$, $Li_2SO_4$, LiF, LiCl, LiBr, LiI, $Li_3PO_4$, $Li_2CO_3$, $Li_2C_2O_4$, $CH_3CO_2Li$ (with varying Li loading, preferably from 1 to 60), Na oxides, $NaNO_3$, $Na_2SO_4$, NaF, NaCl, NaBr, NaI, $Na_3PO_4$, $Na_2CO_3$, $Na_2C_2O_4$, $CH_3CO_2Na$ (with varying Na loading, preferably from 1 to 60), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 60), Rb oxides, $RbNO_3$, $Rb_2SO_4$, RbF, RbCl, RbBr, RbI, $Rb_3PO_4$, $Rb_2CO_3$, $Rb_2C_2O_4$, and $CH_3CO_2Rb$ (with varying Rb loading, preferably from 1 to 60).

**6.** The process according to any one of the preceding claims, wherein the catalyst is selected from the group consisting of xLi-zeolite, xNa-zeolite, xK-zeolite, xRb-zeolite, and xCs-zeolite, preferably xCs-ZSM-5 aluminosilicate zeolite.

**7.** The process according to any one of the preceding claims, wherein the amount of catalysts ranges from 0.5 to 5 g.

**8.** The process according to any one of the preceding claims, wherein the temperature is from 250 to 450 °C.

**Patentansprüche**

**1.** Verfahren zur Herstellung von Glycidol (GLD), umfassend die Schritte:

(a) Zuführen eines Ausgangsmaterials über einen heterogenen Katalysator in einen kontinuierlichen Festbett-Strömungsreaktor,
(b) Erhitzen der Mischung auf eine Temperatur größer als 250 °C, um Glycidol zu erhalten,
(c) Fließen von $N_2$ als ein Trägergas und Glycerin (GL) als eine Beschickung mit einer stündlichen Gesamtgasraumgeschwindigkeit von 1000-2500 $h^{-1}$, und
(d) Sammeln des GLD-Produkts,
wobei das Ausgangsmaterial eine GL-Lösung ist,
wobei der Katalysator mindestens eine Alkalimetallverbindung in einer Menge von 1 bis 60 Gew.-% umfasst, die auf einem Aluminosilikat-Zeolith aufgebracht ist.

**2.** Verfahren nach Anspruch 1, wobei die Konzentration von GL in der Lösung im Bereich von 1 bis 60 Gew.-% liegt.

**3.** Verfahren nach einem der vorhergehenden Ansprüche, wobei die Alkalimetalle ausgewählt sind aus Lithium (Li), Natrium (Na), Kalium (K), Rubidium (Rb), und Cäsium (Cs).

**4.** Verfahren nach einem der vorhergehenden Ansprüche, wobei der Zeolith ausgewählt ist aus der Gruppe bestehend aus ZSM-5, Mordenit, USY, Zeolith L, Ferrierit, Zeolith Beta und Zeolith Y, vorzugsweise ZSM-5.

**5.** Verfahren nach einem der vorhergehenden Ansprüche, wobei die Alkalimetallverbindungen ausgewählt sind aus der Gruppe bestehend aus Cs-Oxiden, CsNOs, $Cs_2SO_4$, CSF, CsCl, CsBr, Csl, $Cs_3PO_4$, $Cs_2COs$, $Cs_2C_2O_4$, $CH_3CO_2Cs$ (mit unterschiedlicher Cs-Beladung, vorzugsweise von 1 bis 60), Li-Oxiden, $LiNO_3$, $Li_2SO_4$, LiF, LiCl, LiBr, Lil, $Li_3PO_4$, $Li_2CO_3$, $Li_2C_2O_4$, $CH_3CO_2Li$ (mit unterschiedlicher Li-Beladung, vorzugsweise von 1 bis 60), Na-Oxiden, $NaNO_3$, $Na_2SO_4$, NaF, NaCl, NaBr, NaI, $Na_3PO_4$, $Na_2CO_3$, $Na_2C_2O_4$, $CH_3CO_2Na$ (mit unterschiedlicher Na-Beladung, vorzugsweise von 1 bis 60), K-Oxiden, $KNO_3$, $K_2SO_4$, KF, KCl, KBr, KI, $K_3PO_4$, $K_2CO_3$, $K_2C_2O_4$, $CH_3CO_2K$ (mit unterschiedlicher K-Beladung, vorzugsweise von 1 bis 60), Rb-Oxiden, $RbNO_3$, $Rb_2SO_4$, RbF, RbCl, RbBr, Rbl, $Rb_3PO_4$, $Rb_2CO_3$, $Rb_2C_2O_4$, und $CH_3CO_2Rb$ (mit unterschiedlicher Rb-Beladung, vorzugsweise von 1 bis 60).

**6.** Verfahren nach einem der vorhergehenden Ansprüche, wobei der Katalysator ausgewählt ist aus der Gruppe bestehend aus xLi-Zeolith, xNa-Zeolith, xK-Zeolith, xRb-Zeolith und xCs-Zeolith, vorzugsweise xCs-ZSM-5-Aluminosilikat-Zeolith.

**7.** Verfahren nach einem der vorhergehenden Ansprüche, wobei die Menge der Katalysatoren im Bereich von 0,5 bis 5 g liegt.

**8.** Verfahren nach einem der vorangehenden Ansprüche, wobei die Temperatur von 250 bis 450 °C liegt.

**Revendications**

**1.** Procédé de production du glycidol (GLD) comprenant les étapes:

(a) introduire un produit de départ sur un catalyseur hétérogène dans un réacteur à flux continu à lit fixe,
(b) chauffer ledit mélange à une température supérieure à 250°C pour obtenir du glycidol,
(c) faire circuler du $N_2$ comme gaz porteur et du glycérol (GL) comme alimentation avec une vitesse spatiale horaire totale de gaz de 1000 à 2500 $h^{-1}$, et

(d) recueillir le produit GLD,

dans lequel le produit de départ est une solution de GL,

dans lequel le catalyseur comprend au moins un composé de métal alcalin en une quantité de 1 à 60 % en poids supporté sur une zéolite d'aluminosilicate.

2. Procédé selon la revendication 1, dans lequel la concentration de GL dans la solution est dans la plage de 1 à 60 % en poids.

3. Procédé selon l'une quelconque des revendications précédentes, dans lequel les métaux alcalins sont choisis parmi le lithium (Li), le sodium (Na), le potassium (K), le rubidium (Rb) et le césium (Cs).

4. Procédé selon l'une quelconque des revendications précédentes, dans lequel la zéolite est choisie dans le groupe consistant en ZSM-5, mordénite, USY, zéolite L, ferriérite, zéolite bêta et zéolite Y, de préférence ZSM-5.

5. Procédé selon l'une quelconque des revendications précédentes, dans lequel les composés de métal alcalin sont choisis dans le groupe consistant en les oxydes de Cs, $CsNO_3$, $Cs_2SO_4$, $CsF$, $CsCl$, $CsBr$, $CsI$, $Cs_3PO_4$, $Cs_2CO_3$, $Cs_2C_2O_4$, $CH_3CO_2Cs$ (avec une charge de Cs variable, de préférence de 1 à 60), les oxydes de Li, $LiNO_3$, $Li_2SO_4$, $LiF$, $LiCl$, $LiBr$, $LiI$, $Li_3PO_4$, $Li_2CO_3$, $Li_2C_2O_4$, $CH_3CO_2Li$ (avec une charge de Li variable, de préférence de 1 à 60), les oxydes de Na, $NaNO_3$, $Na_2SO_4$, $NaF$, $NaCl$, $NaBr$, $NaI$, $Na_3PO_4$, $Na_2CO_3$, $Na_2C_2O_4$, $CH_3CO_2Na$ (avec une charge de Na variable, de préférence de 1 à 60), les oxydes de K, $KNO_3$, $K_2SO_4$, $KF$, $KCl$, $KBr$, $KI$, $K_3PO_4$, $K_2CO_3$, $K_2C_2O_4$, $CH_3CO_2K$ (avec une charge de K variable, de préférence de 1 à 60), les oxydes de Rb, $RbNO_3$, $Rb_2SO_4$, $RbF$, $RbCl$, $RbBr$, $RbI$, $Rb_3PO_4$, $Rb_2CO_3$, $Rb_2C_2O_4$ et $CH_3CO_2Rb$ (avec une charge de Rb variable, de préférence de 1 à 60).

6. Procédé selon l'une quelconque des revendications précédentes, dans lequel le catalyseur est choisi dans le groupe consistant en zéolite xLi, zéolite xNa, zéolite xK, zéolite xRb et zéolite xCs, de préférence la zéolite d'aluminosilicate xCs-ZSM-5.

7. Procédé selon l'une quelconque des revendications précédentes, dans lequel la quantité de catalyseurs va de 0,5 à 5 g.

8. Procédé selon l'une quelconque des revendications précédentes, dans lequel la température est de 250 à 450°C.

# Fig. 1

# Fig. 2

# Fig. 3

**(a)**

**(b)**

# Fig. 4

**Fig. 5**

# Fig. 6

(a)

(b)

**Fig. 7**

**Fig. 8**

(a)

(b)

**Fig. 9**

**Fig. 10**

**Fig. 11**

# Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130090497 A1, A. Pienaar, L. Wilson, M. Stockenhuber

- US 20140179936 A1, A. Pienaar, L. Wilson, M. Stockenhuber

**Non-patent literature cited in the description**

- **Y. ENDAH ; M. KIM ; J. CHOI ; J. JAE ; S. LEE ; H. LEE.** *Catal. Today,* 2017, vol. 293-294, 136-141

- **S. KONDAWAR ; C. PATIL ; C. RODE.** *ACS Sustain. Chem. Eng.,* 2017, vol. 5, 1763-1774
- **RAMLI ZAINAB et al.** *Jurnal Teknologi,* 01 June 2005, vol. 42 (C), 43-55